# EUROPEAN PATENT SPECIFICATION

(11) **EP 0 983 270 B1**
(45) Date of publication and mention of the grant of the patent: **18.02.2004**
(21) Application number: 98919929.4
(22) Date of filing: 27.04.1998
(51) Int. Cl.: C07D 471/04

(54) **PROCESS FOR PREPARING 6-O-MONOESTERS OF CASTANOSPERMINE**
VERFAHREN ZUR HERSTELLUNG VON 6-0-MONOESTERN VON CASTANOSPERMINE
PROCEDE DE PREPARATION DE 6-O-MONOESTERS DE CASTANOSPERMINE

(30) Priority: 22.05.1997 US 861874
(43) Date of publication of application: 08.03.2000
(73) Proprietor: Aventis Pharmaceuticals Inc., Bridgewater, NJ 08807-0800 (US)
(72) Inventor: GORALSKI, Christian, T., Midland, MI 48642 (US); STOLZ-DUNN, Sandra, Midland, MI 48640 (US); HITT, James, E., Midland, MI 48642 (US); LOUKS, David, H., Saginaw, MI 48609 (US); SCHERZER, Brian, D., Saginaw, MI 48603 (US); NITZ, Mark, A., Sanford, MI 48657 (US); SMIT, Mark, R., Midland, MI 48640 (US)
(74) Representative: Minoja, Fabrizio, Dr.
(86) International application number: PCT/US1998/008471
(87) International publication number: WO 1998/052947

(56) References cited:
- R.FURNEAUX ET AL.: "THE CHEMISTRY OF CASTANOSPERMINE,PART 1:SYNTHETIC MODIFICATIONS AT C-6." TETRAHEDRON., vol. 50, no. 7, 1994, pages 2131-2160, XP002074670 OXFORD GB cited in the application
- WAYNE K.ANDERSON ET AL.: "A FACILE SELECTIVE ACYLATION OF CASTANOSPERMINE." TETRAHEDRON LETTERS., vol. 31, no. 2, 1990, pages 169-170, XP002074671 OXFORD GB cited in the application

## Description

Castanospermine, [1*S*-(1α,6β,7α,8β,8aβ)]-octahydro-1,6,7,8-indolizinetetrol, is an indolizidine alkaloid with the following structure: 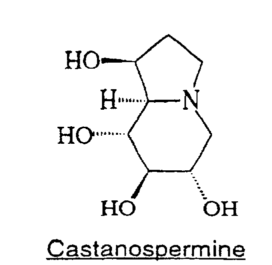 It is isolated from *Castanospermum australe* as described by L.D. Hohenshutz, et al., *Phytochemistry,* 20, 811 (1981) and from *Alexa leiopetala* as described by R.J. Nash, et al., *Phytochemistry,* 27, 1403 (1988). Certain esters of castanospermine have been found to be useful in the treatment of various retroviral infections, diabetes and in the inhibition of tumor metastasis as disclosed respectively in United States Patent No. 5,004,746 issued April 2, 1991, United States Patent No. 5,017,563 issued May 21, 1991 and United States Patent No. 4,952,585 issued August 28, 1990, the disclosures of which are incorporated herein by reference. More specifically, 6-0-butyrylcastanospermine • HCl, which has the following structure: 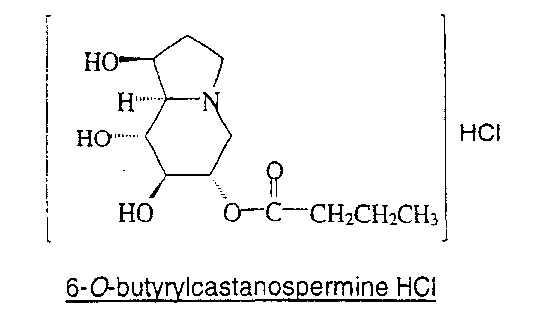 has been of particular interest.

interest in developing a short and efficient synthesis of the 6-*O*-monoesters of castanospermine has been substantial. However, with four relatively similar secondary alcohols present on castanospermine, selective mono-acylation at the 6-position has presented a significant challenge. The original synthesis of the 6-*O*-monoesters of castanospermine, as disclosed by Sunkara and Liu in U.S. Patent No. 4,952,585, involved treating castanospermine with an excess of an acid chloride in pyridine at 0°C followed by three days of stirring. An aqueous extraction followed by purification of the complex mixture by radial chromatography provided the 6-*O*-monoester in poor yield.

A subsequent synthesis of the 6-*O*-monoesters of castanospermine, disclosed by P.S. Liu, et al. *Tetrahedron Lett.,* 31 (20), 2829 (1990), utilized selective protection and deprotection of the hydroxyl groups with mono-acylation of the 6-hydroxyl group. The overall process resulted in improved yields, however a total of five steps was required starting from castanospermine.

A one-pot procedure for the preparation of 6-monoesters of castanospermine was reported by W.K. Anderson, et al., *Tetrahedron Lett.* 31(2), 169 (1990) wherein castanospermine was treated with dibutyltin oxide in methanol. The reaction was heated to reflux, then cooled and treated with an acid chloride and triethylamine to provide the desired free base of the 6-monoester of castanospermine in yields ranging from 18% to 44% after flash chromatography.

An improved one-pot procedure for the preparation of 6-monoesters of castanospermine was reported by P.C. Tyler, et al., *Tetrahedron*, 50(7), 2131 (1994) wherein castanospermine was treated with bis(tributyltin) oxide in toluene. The reaction was heated to reflux with removal of water in order to drive the reaction to completion. After 2 hours, the reaction was cooled to - 10°C and treated with an acid chloride to provide the desired 6-monoester of castanospermine. Using this procedure, the free bases of 6-*O*-benzoylcastanospermine and 6-*O*-butyrylcastanospermine were prepared in yields of 94% and 83%, respectively.

However, it has been found that as the size of the reaction is increased following the procedure of Tyer et al., the time required to drive the formation of the tributyltin ether to completion through removal of water is also increased. It has further been found that by replacing toluene with xylene as the solvent in the process of Tyler et al., the time required to drive the reaction to completion by removal of water is substantially decreased.

### SUMMARY OF THE INVENTION

The present invention relates to a process for preparing a compound of formula (I): 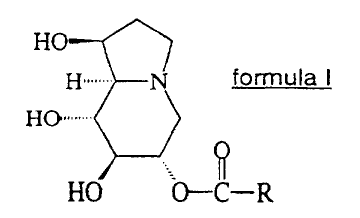 wherein R is C₁-C₁₀ alkyl, phenyl or substituted phenyl, comprising treating a compound of formula (II): 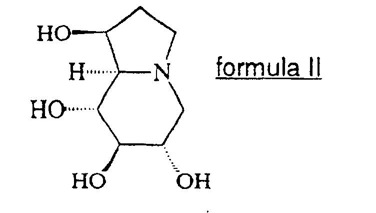 with bis(tributyltin) oxide in an organic solvent selected from the group consisting of *o*-xylene, *m*-xylene, *p*-xylene and mixed xylenes; and subsequently treating the reaction mixture with a compound of formula (III): 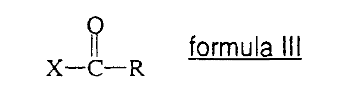 wherein X is halogen and R is defined as above.

The invention further relates to a process for preparing a compound of formula (IV): 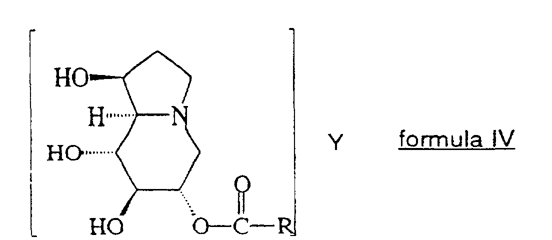 wherein R is C₁-C₁₀ alkyl, phenyl or substituted phenyl; and Y is an acid selected from the group consisting of hydrogen bromide, hydrogen chloride, sulfuric acid, phosphoric acid, nitric acid, formic acid, acetic acid, propionic acid, succinic acid, glycolic acid, lactic acid, malic acid, tartaric acid, citric acid, ascorbic acid, α-ketoglutaric acid, glutamic acid, aspartic acid, maleic acid, hydroxymaleic acid, pyruvic acid, phenylacetic acid, benzoic acid, *p*-aminobenzoic acid, anthranilic acid, *p*-hydroxybenzoic acid, salicyclic acid, hydroxyethanesulfonic acid, ethylenesulfonic acid, halobenzenesulfonic acid, toluenesulfonic acid, naphthalenesulfonic acid, methanesulfonic acid and sulfanilic acid, comprising:
a) treating a compound of formula (II): 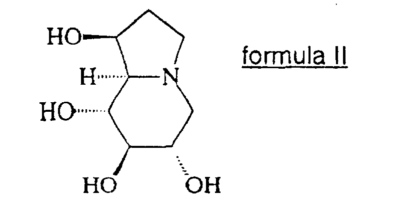 with bis(tributyltin) oxide in an organic solvent selected from the group consisting of *o*-xylene, *m*-xylene, *p*-xylene and mixed xylenes;
   and subsequently treating the reaction mixture with a compound of formula (III): 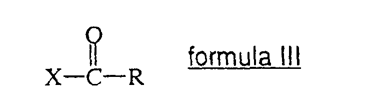 wherein X is halogen and R is defined as above; and
b) subsequently treating the reaction mixture with a suitable organic solvent and an acid of formula Y defined above.

### DETAILED DESCRIPTION OF THE INVENTION

As used herein the term "C₁-C₄ alkyl" refers to a saturated straight or branched chain alkyl radical of one to four carbon atoms. Included within the scope of this term are methyl, ethyl, n-propyl, isopropyl, n-butyl, isobutyl and the like. The term "C₁-C₆ alkyl" refers to a branched or straight chained, or cyclic alkyl radical containing from 1-6 carbon atoms, such as methyl, ethyl, *n*-propyl, isopropyl, *n*-butyl, isobutyl, *t*-butyl, *n*-pentyl, cyclopentyl, *n*-hexyl, cyclohexyl, and the like. The term "C₁-C₁₀ alkyl" refers to a saturated straight or branched chain or cyclic alkyl radical of one to ten carbon atoms, including methyl, ethyl, propyl, isopropyl, *n*-butyl, isobutyl, t-butyl, n-pentyl, isopentyl, hexyl, 2,3-dimethyl-2-butyl, heptyl, 2,2-dimethyl-3-pentyl, 2-methyl-2-hexyl, octyl, 4-methyl-3-heptyl, nonyl, decyl and the like.

The term "phenyl" refers the phenyl functionality of the formula:

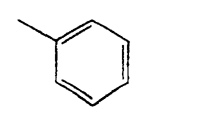

The term "substituted phenyl" refers the substituted phenyl functionality of the formula: 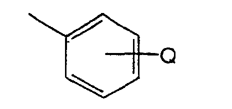 wherein Q is a C₁-C₄ alkyl, nitro or halo which can be located at the ortho, meta or para position on the ring.

The terms "halo", "halogen" or "halide" refers to a fluorine, chlorine, bromine or iodine atom.

As used herein the term "ethanol 3C" refers to ethanol which has been denatured with 5% isopropanol.

The term "pharmaceutically acceptable salt" refers to those salts that are not substantially toxic at the dosage administered to achieve the desired effect and do not independently possess significant pharmacological activity. The salts included within the scope of this term are hydrobromide, hydrochloride, sulfuric, phosphoric, nitric, formic, acetic, propionic, succinic, glycolic, lactic, malic, tartaric, citric, ascorbic, α-ketoglutaric, glutamic, aspartic, maleic, hydroxymaleic, pyruvic, phenylacetic, benzoic, *p*-aminobenzoic, anthranilic, *p*-hydroxybenzoic, salicyclic, hydroxyethanesulfonic, ethylenesulfonic, halobenzenesulfonic, toluenesulfonic, naphthalenesulfonic, methanesulfonic, sulfanilic, and the like. Such salts can exist in either a hydrated or substantially anhydrous form.

The pharmaceutically acceptable salts of formula (I) are prepared from the corresponding acids disclosed immediately above, such as hydrogen bromide, hydrogen chloride, sulfuric acid, phosphoric acid, nitric acid, formic acid, acetic acid, propionic acid, succinic acid, glycolic acid, lactic acid, malic acid, tartaric acid, citric acid, ascorbic acid, α-ketoglutaric acid, glutamic acid, aspartic acid, maleic acid, hydroxymaleic acid, pyruvic acid, phenylacetic acid, benzoic acid, *p*-aminobenzoic acid, anthranilic acid, *p*-hydroxybenzoic acid, salicyclic acid, hydroxyethanesulfonic acid, ethylenesulfonic acid, halobenzenesulfonic acid, toluenesulfonic acid, naphthalenesulfonic acid, methanesulfonic acid, sulfanilic acid and the like. The preferred suitable acid is hydrogen chloride.

The compounds of formula (I) and (IV) can be prepared as described in Scheme 1. All substituents, unless otherwise indicated, are previously defined. The reagents and starting materials are readily available to one of ordinary skill in the art.

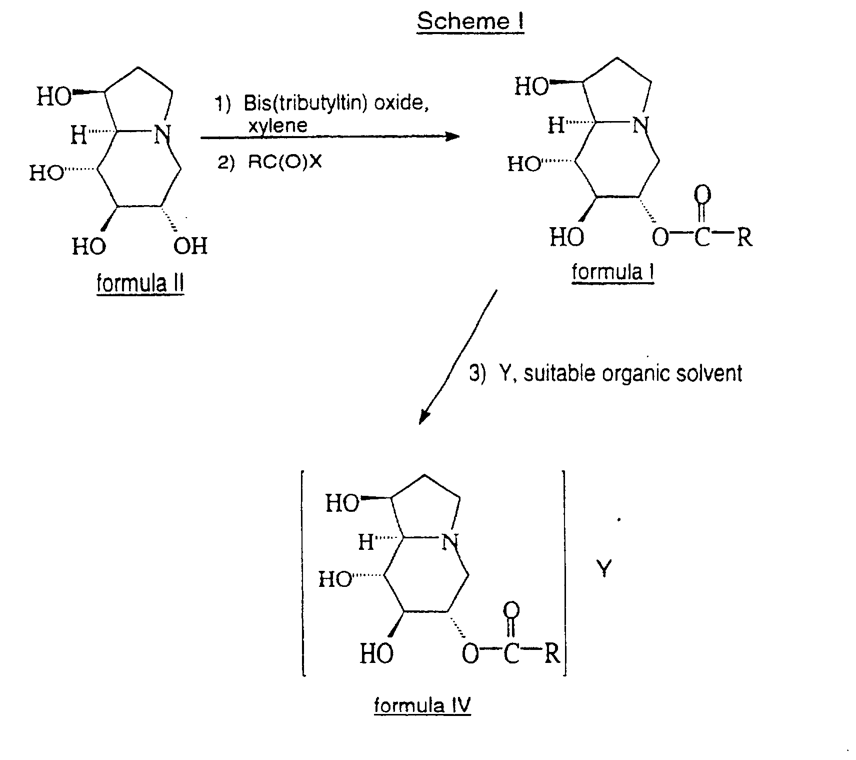

In Scheme I, step 1, under an inert atmosphere, such as nitrogen, castanospermine (formula II) is combined with a suitable organic solvent selected from the group consisting of *o*-xylene, *m*-xylene, *p*-xylene and mixed xylenes, and 1.5 to 2.5 equivalents of bis(tributyltin) oxide, with 2.2 equivalents of bis(tributyltin) oxide being preferred. The mixture is heated at reflux, and, the by-product water is collected via a suitable apparatus, such as a Dean-Stark trap in order to drive the reaction to completion. The mixture is heated at reflux for 0.5 to 6 hours, with 1 to 2 hours being preferred. In step 2 of Scheme I, the mixture is then cooled to -20°C to -10°C with -15°C being preferred. To the cooled mixture is added 1.5 to 2.0 equivalents of an acid chloride of formula III, with 1.8 equivalents being preferred. 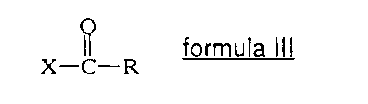 The acid chloride of formula III is added at such a rate that the temperature of the mixture is maintained between -20°C to 20°C, with -17°C to 5°C being preferred. Examples of acid chlorides of formula III include acetyl chloride, propionyl chloride, butyryl chloride, isobutyryl chloride, trimethylacetyl chloride, valeryl chloride, isovaleryl chloride, *tert* butylacetyl chloride, hexanoyl chloride, heptanoyl chloride, 2-ethylhexanoyl chloride, octanoyl chloride, nonanoyl chloride, decanoyl chloride, benzoyl chloride, 4-butylbenzoyl chloride, *p*-*tert*-butylbenzoyl chloride, o-, *m-* or *p*- chlorobenzoyl chloride, *m*- or *p*-nitrobenzoyl chloride, *o-, m*- or *p*-toluoyl chloride and the like. Butyryl chloride is the preferred acid chloride of formula III. After addition of the acid chloride is complete, the mixture is allowed to warm to 20°C and is then diluted with a suitable organic solvent, such as ethanol 3C (ethanol denatured with 5% isopropanol). Isolation of the free base of formula I from the reaction mixture is somewhat difficult, thus it is preferred that the compound of formula I be directly converted to the compound of formula IV without intermediate isolation of the compound of formula I, by treatment with an acid of formula Y, which has been defined previously herein. For example, in step 3 of Scheme I, the compound of formula I in the suitable organic solvent, such as a mixture of xylenes and ethanol 3C, is made strongly acidic by treatment with an acid of formula Y, such as anhydrous hydrogen chloride. The resulting compound of formula IV is isolated and purified by techniques well known by one of ordinary skill in the art, such as crystallization. For example, the solution may be seeded with several crystals of the desired compound of formula IV in order to initiate crystallization. The resulting slurry is then cooled with an ice bath and the solids are collected by filtration, for example through a centrifugal filter. The solids are then washed with a suitable organic solvent, such as heptanes, to remove residual tributyltin compounds, and air and vacuum dried to provide the compound of formula IV.

The following examples present typical syntheses as described in Scheme I. These examples are understood to be illustrative only and are not intended to limit the scope of the present invention in any way. It is further understood that the apparatus necessary for carrying out the present invention on the chosen scale is readily determined by one of ordinary skill in the art, with the following exception. The removal of the by-product water in step 1 of Scheme I may necessarily be enhanced as the size of the reaction is increased. This can be accomplished by the use of a rectification column during reflux, by increasing the heat transfer area available during reflux, by sparging an inert gas, such as nitrogen, through the refluxing solution, by sparging vapor phase solvent through the refluxing solution, by sending back solvent to the refluxing solution that has a water content much less than the saturation level, using a technique such as drying with a drying agent, or by other methods that will shift the equilibrium of the desired reaction to favor the desired product via removal of water from the reaction solution.

As used herein, the following terms have the indicated meanings: "kg" refers to kilograms: "m²/g" refers to square meters per gram and is used as a measurement of particle surface area; "lbs" refers to pounds; "g" refers to grams; "mg" refers to milligrams; "µg" refers to micrograms; "ppm" refers to parts per million; "mmol" refers to millimoles; "mL" refers to milliliters; "cm" refers to centimeters; "L" refers to liters; "°C" refers to degrees Celsius; "°F" refers to degrees Fahrenheit; "mm Hg" refers to millimeters of mercury; "rpm" refers to revolutions per minute; "R_{f}" refers to retention factor; "bp" refers to boiling point; "mp" refers to melting point; "dec" refers to decomposition; "M" refers to molar; "mM" refers to millimolar; "µM" refers to micromolar; "nM" refers to nanomolar; "µL" refers to microliters; "HPLC" refers to high performance liquid chromatography; "eq" refers to equivalents; "h" refers to hours; "N" refers to normal; and "Rₜ" refers to retention time.

### Example 1

### General Procedure for Preparing 6-O-Butyrylcastanosopermine [1S-(1α,6β,7α,8β,8aβ)]-octahydro-1,6,7,8-indolizinetetrol 6-butanoate HCl, Two Liter Scale, with Xylene as Solvent.

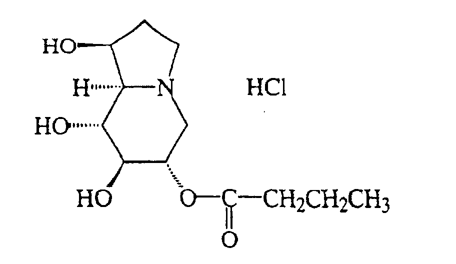

Scheme I, step 1; A 2 liter, three-neck flask equipped with a mechanical stirrer, thermometer and a Dean-Stark trap fitted with a condenser bearing a nitrogen bubbler is charged with castanospermine (20.00 g, 0.106 mol), bis(tributyltin) oxide (115 mL) and xylene (600 mL, mixed isomers). The mixture is heated to reflux and water is collected in the Dean-Stark trap. After 20 minutes, the reaction mixture becomes homogeneous and after one hour and 5 minutes the heating is stopped. Approximately 2 mL of water is collected. The reaction is allowed to cool to room temperature.

Scheme I, step 2; The Dean-Stark trap is removed from the above reaction and is replaced with a Claisen adapter fitted with a nitrogen bubbler and a pressure equalizing addition funnel. The reaction mixture is cooled to -17°C and butyryl chloride (20.20 g, 0.190 mol) is added slowly over 10 minutes, maintaining the temperature between -17°C and -5°C. The reaction mixture is then allowed to warm to 20°C.

Scheme I, step 3; The above reaction mixture is diluted with ethanol 3C (600 mL, ethanol denatured with 5% isopropanol) and treated with anhydrous hydrogen chloride (14.13 g). The reaction temperature rises from 20°C to 32°C during the hydrogen chloride addition. The reaction mixture is seeded with several crystals of 6-*O*-butyrylcastanospermine hydrochloride to produce a slurry of a white solid. The slurry is allowed to stir at room temperature overnight and then is cooled with an ice bath and stirred for 2 hours. The white solid is collected by filtration, air dried, washed with heptane (100 mL, mixed isomers), air dried for 2 hours and dried under vacuum at room temperature for 2 hours to provide the title compound (25.14 g, 80.2%).

**Table I.**

| Results of 11 Experiments for Preparing 6-*O*-Butyrylcastanospermine HCl in Xylene Following the General Procedure Described in Example 1. | | | | | |
|---|---|---|---|---|---|
| Castanospermine g | BTBTO¹ (mL), g | Xylene mL, (g) | Reaction Time hours | Yield % | Castanospermine % |
| 20.00 | (115) | 600 | 1.1 | 80.2 | 0.56 |
| 40.00 | (230) | 525 | 1.5 | 81.5 | 0.42 |
| 20.00 | (54) | 600 | 2.0 | 47.5 | ------ |
| 20.00 | (81) | 600 | 2.5 | 77.7 | 0.51 |
| 20.00 | (94.5) | 600 | 2.0 | 79.4 | 0.44 |
| 20.00 | (81) | 600 | 2.75 | 69.6 | ------ |
| 20.00 | (95) | 600 | 2.0 | 74.0 | 1.44 |
| 20.25 | 137.7 | (222.7) | 2.0 | 66.8 | 0.54 |
| 20.25 | 137.7 | (222.7) | 2.0 | 69.5 | 1.03 |
| 20.25 | 137.7 | (222.7) | 2.0 | 69.3 | 1.06 |
| 20.00 | 132.3 | (522.3) | 2.0 | 77.4 | 0.40 |

| | | | | | |
|---|---|---|---|---|---|
| ¹BTBTO refers to bis(tributyltin) oxide. | | | | | |

### Example 2

### General Procedure for Preparinq 6-O-Butyrylcastanospermine [1S-(1α,6β,7α,8β,8aβ)]-octahydro-1,6,7,8-indolizinetetrol 6-butanoate HCl Two Liter Scale with Toluene as Solvent.

Scheme I, step 1; A 2 liter, three-neck flask equipped with a mechanical stirrer, thermometer and a Dean-Stark trap fitted with a condenser bearing a nitrogen bubbler is charged with castanospermine (20.00 g, 0.106 mol), bis(tributyltin) oxide (115 mL) and toluene (600 mL). The mixture is heated to reflux and water is collected in the Dean-Stark trap. After 3.33 hours the heating is stopped and the reaction mixture is cooled to 20°C.

Scheme I, step 2; The Dean-Stark trap is removed from the above reaction and is replaced with a Claisen adapter fitted.with a nitrogen bubbler and a pressure equalizing addition funnel. The reaction mixture is cooled to -13°C and butyryl chloride (20.20 g, 0.190 mol) is added slowly over 10 minutes, maintaining the temperature between -13°C and -8°C. The reaction mixture is then allowed to warm to room temperature.

Scheme I, step 3; The above reaction mixture is diluted with ethanol 3C (650 mL, ethanol denatured with 5% isopropanol) and treated with anhydrous hydrogen chloride (approximately 18.00 g, 0.494 mol). The reaction temperature rises from 18°C to 30°C during the hydrogen chloride addition. The reaction mixture is seeded with several crystals of 6-*O*-butyrylcastanospermine hydrochloride to produce a slurry of a white solid. The slurry is allowed to stir at room temperature overnight and then is cooled with an ice bath and stirred for 2 hours. The white solid is collected by filtration, air dried, washed with heptane (100 mL, mixed isomers), air dried and dried under vacuum at room temperature to provide the title compound (24.26 g, 77.4%).

### Example 3

### General Procedure for Preparing 6-O-Butyrylcastanospermine [1S-(1α,6β,7α,8β,8aβ]-octahydro-1,6,7,8-indolizinetetrol 6-butanoate HCl. Pilot Plant Scale. with Xylene as Solvent.

Scheme I, step 1; A 200 gallon (757 liters) batch reactor fitted with a Dean-Stark trap is charged with bis(tributyltin) oxide (326 lbs, 147.9 kg), mixed xylenes (528 lbs, 239.5 kg) and castanospermine (48 lbs, 21.8 kg) with an agitator speed of 60 rpm and jacket temperature set at 25°C. The condenser temperature is set at 10°C and the mixture is then heated by increasing the jacket temperature of the reactor at a rate of 5°C/min up to reflux temperature of 175°C. After 6 hours, 70 lbs (31.8 kg) of xylene/water are collected from the trap. The condenser is then set at -25°C and after 1,5 hours, 45 lbs (20.4 kg) of xylene/water are collected from the trap. The reaction is then cooled to 25°C.

The progress of the above stannylation reaction is evaluated by the following procedure; A 1.7 g sample of the above reaction mixture is transferred to a clean, dry, 7-dram vial. To this is added benzoyl chloride (0.14-0.16 g), the vial is sealed and the reaction mixture is shaken. A precipitate of the benzoyl derivative of castanospermine (6-*O*-benzoylcastanospermine) quickly forms. The vial is then nearly filled with water, adjusted to pH 2.0 with phosphoric acid, a small amount of mixed heptanes is added, and the mixture is vigorously shaken. The layers are then allowed to separate. Two disposable pipette volumes of the aqueous layer are transferred to a clean, 7-dram vial and the vial is nearly filled with pH 2.0 water. The mixture is shaken and the resulting solution is analyzed by High Performance Liquid Chromatography under standard conditions readily determined by one of ordinary skill in the art to determine whether the reaction has gone to completion.

Scheme I, step 2; The reaction mixture is then cooled by setting the jacket temperature of the reactor at -15°C, and with the agitator speed still at 60 rpm, butyryl chloride (48 Ibs, 21.8 kg) is added at such a rate that the reaction temperature is maintained below 10°C. After one hour the jacket temperature is set at 25°C and the reaction mixture is stirred for an additional hour.

Scheme I, step 3; The reaction mixture is then filtered through a 0.2 micron retention polypropylene filter into a 300 gallon (1135.5 liters) reactor to which a solution of ethanol 3C (384 lbs, 174.2 kg) and anhydrous HCl (16.5 lbs (7.5 kg) bubbled-into the ethanol 3C) has already been charged. The original 200 gallon (757 liters) reactor and transfer line are flushed with mixed xylenes (123 lbs, 55.8 kg) into the 300 gallon (1135.5 liters) reactor. The agitator speed is set at 30 rpm and the jacket temperature is set at 25°C. Anhydrous HCl (12 lbs, 5.4 kg) is then bubbled into the reaction mixture until a pH of less then 3.0 is obtained (4 hours). The jacket temperature is then set at -10°C and the mixture is stirred for 4 hours. The title compound is then isolated by centrifugation and the product is washed with mixed heptanes (192 Ibs, 87.1 kg). The title compound is dried under vacuum (100 mmHg) at a temperature of less than 35°C to provide the dry title compound.

**Table II.**

| Results of 21 Experiments for Preparing 6-*O*-Butyrylcastanospermine HCl in Xylene Following the General Procedure Described in Example 3. | | | | | |
|---|---|---|---|---|---|
| Castanospermine lbs, kg | BTBTO¹ lbs, kg | BuCl² lbs, kg | Xylene lbs, kg | Yield³ lbs, kg | Yield³ % |
| 30.0, 13.6 | 204.0, 92.5 | 30.0, 13.6 | 330.0, 149.7 | 34.8, 15.8 | 74.1 |
| 40.0,18.1 | 272.0,123.4 | 40.0, 18.1 | 442.0, 200.5 | 53.1,24.1 | 85.0 |
| 49.5, 22.5 | 333.5, 151.3 | 49.0, 22.2 | 609.0, 276.2 | 60.0, 29.9 | 85.3 |
| 50.5,22.9 | 341.0, 154.7 | 50.0, 22.7 | 550.0, 249.5 | 57.3, 26.0 | 72.6 |
| 40.0, 18.1 | 272.2, 123.5 | 40.0, 18.1 - | 440.3, 199.7 | 39.2, 17.8 | 62.7 |
| 48.0,21.8 | 326.0, 147.9 | 48.0,21.8 | 528.0, 239.5 | 65.3, 29.6 | 87.1 |
| 48.0, 21.8 | 326.0, 147.9 | 48.0,21.8 | 528.0, 239.5 | 61.0,27.7 | 81.3 |
| 48.0, 21.8 | 326.0, 147.9 | 48.0,21.8 | 528.0, 239.5 | 56.8, 25.8 | 75.8 |
| 47.7, 21.6 | 320.7,145.5 | 47.0,21.3 | 517.1,234.6 | 60.5,27.4 | 81.1 |
| 52.0,23.6 | 354.4, 160.8 | 52.0, 23.6 | 572.0, 259.5 | 68.9, 31.3 | 84.8 |
| 52.6, 23.9 | 354.5, 160.8 | 52.0,23.6 | 572.5, 259.7 | 61.9,28.1 | 75.3 |
| 51.3, 23.3 | 362.4, 164.4 | 52.0,23.6 | 572.5,259.7 | 71.9,32.6 | 89.7 |
| 37.5, 17.0 | 255.5, 115.9 | 37.5, 17.0 | 532.0, 241.3 | 50.3, 22.8 | 85.8 |
| 37.2, 16.9 | 255.0, 115.7 | 37.5, 17. 0 | 412.5, 187.1 | 46.7, 21.2 | 80.4 |
| 48.0, 21.8 | 326.3, 148.0 | 48.0,21.8 | 529.9, 240.4 | 53.3, 24.2 | 71.1 |
| 52.0,23.6 | 326.9, 148.3 | 48.0, 21.8 | 528.1,239.6 | 56.7,25.7 | 69.8 |
| 48.0, 21.8 | 327.0, 148.3 | 48.0,21.8 | 528.0; 239.5 | 63.0,28.6 | 84.0 |
| 48.1,21.8 | 326.5, 148.1 | 48.0,21.8 | 528.0,239.5 | 59.4,26.9 | 79.0 |
| 47.8,21.7 | 326.5, 148.1 | 48.0,21.8 | 528.0, 239.5 | 66.2,30.0 | 88.5 |
| 55.0, 25.0 | 374.7, 170.0 | 55.0,25.0 | 605.7, 274.8 | 69.7, 31.6 | 81.1 |
| 55.0,25.0 | 374.0, 169.7 | 55.0,25.0 | 605.0, 274.4 | 81.5,37.0 | 94.8 |

| | | | | | |
|---|---|---|---|---|---|
| ¹BTBTO refers to bis(tributyltin) oxide. | | | | | |
| ²BuCl refers to butyryl chloride. | | | | | |
| ³Yield of 6-*O*-butyrylcastanospermine HCl | | | | | |

## Claims

1. A process for preparing a compound of the formula: 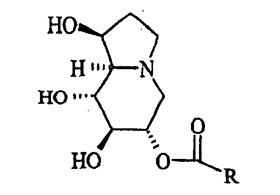 wherein R is C₁-C₁₀ alkyl, phenyl or phenyl substituted with C₁-C₄ alkyl, nitro or a halogen atom selected from fluorine, chlorine, bromine or iodine located at the ortho, meta or para position on the ring,
comprising treating a compound of the formula: 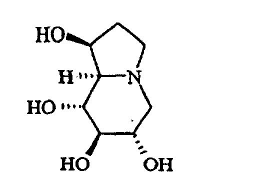 with bis(tributyltin) oxide in an organic solvent selected from the group consisting of *o*-xylene, *m*-xylene, *p*-xylene and mixed xylenes;
and subsequently treating the reaction mixture with a compound of the formula: 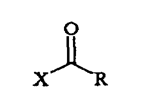 wherein X is halogen and R is defined as above.

2. A process for preparing a compound of the formula: 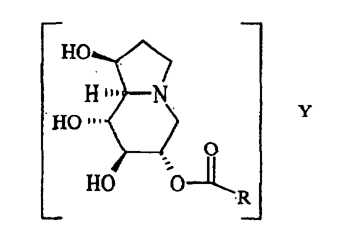 wherein R is C₁-C₁₀ alkyl, phenyl or phenyl substituted with C₁-C₄ alkyl, nitro or a halogen atom selected from fluorine, chlorine, bromine or iodine located at the ortho, meta. or para position on the ring; and Y. is an acid selected from the group consisting of hydrogen bromide, hydrogen chloride, sulfuric acid, phosphoric acid, nitric acid, formic acid, acetic acid, propionic acid, succinic acid, glycolic acid, lactic acid, malic acid, tartaric acid; citric acid, ascorbic acid, α-ketoglutaric acid, glutamic acid, aspartic acid, maleic acid, hydroxymaleic acid, pyruvic acid, phenylacetic acid, benzoic acid, *p*-aminobenzoic acid, anthranilic acid, *p*-hydroxybenzoic acid, salicyclic acid, hydroxyethanesulfonic acid, ethylenesulfonic acid, halobenzenesulfonic acid, toluenesulfonic acid, naphthalenesulfonic acid, methanesulfonic acid and sulfanilic acid, comprising;
a) treating a compound of the formula: 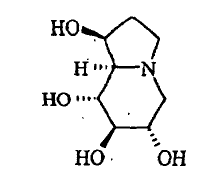 with bis(tributyltin) oxide and with a compound of the formula: 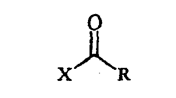 in accordance with the procedures as set forth in claim 1, wherein X is halogen and R is defined as above; and
b). subsequently treating the reaction mixture with a suitable organic solvent and an acid of formula Y defined above.

3. The process according to either claim 1 or claim 2 wherein R is C₁-C₄ alkyl or phenyl.

4. The process according to either claim 1 or claim 2 wherein R is - CH₂CH₂CH₃.

5. The process according to claim 4 wherein the organic solvent is mixed xylenes.

6. The process according to claim 5 wherein Y is hydrogen chloride.

7. the process according to claim 6 wherein X is Cl.

8. The process according to claim 7 wherein the mixture comprising the compound of said formula, 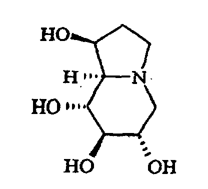 said bis(tributyltin)oxide and said mixed xylenes, is heated at reflux for 1 to 6 hours.

9. The process according to claim 8 wherein the mixture is heated at reflux for 1 to 2 hours.

10. The process according to claim 9 wherein the suitable organic solvent is ethanol 3C.

## Patentansprüche

1. Verfahren zur Herstellung einer Verbindung der Formel: 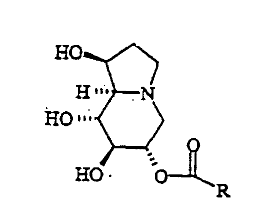 worin R C₁-C₁₀ Alkyl, Phenyl oder Phenyl, substituiert mit C₁-C₄ Alkyl, Nitro oder einem Halogenatom, ausgewählt unter Fluor, Chlor, Brom oder Jod, in ortho-, meta- oder para-Stellung an dem Ring bedeutet, umfassend die Behandlung einer Verbindung der Formel 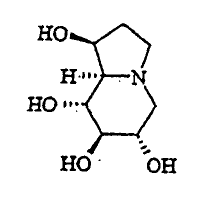 mit Bis(tributylzinn)oxid in einem organischen Lösungsmittel, ausgewählt unter o-Xylol, m-Xylol, p-Xylol und gemischten Xylolen;
und die anschließende Behandlung der Reaktionsmischung mit einer Verbindung der Formel: 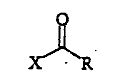 worin X Halogen bedeutet und R wie vorstehend definiert ist.

2. Verfahren zur Herstellung einer Verbindung der Formel: 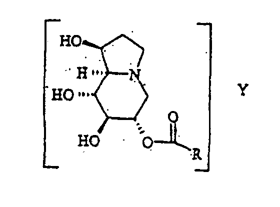 worin R C₁-C₁₀ Alkyl, Phenyl oder Phenyl, substituiert mit C₁-C₄ Alkyl, Nitro oder einem Halogenatom, ausgewählt unter Fluor, Chlor, Brom oder Jod, in ortho-, meta- oder para-Stellung an dem Ring, bedeutet, und Y eine Säure ist, ausgewählt unter Bromwasserstoff, Chlorwasserstoff, Schwefelsäure, Phosphorsäure, Salpetersäure, Ameisensäure, Essigsäure, Propionsäure, Bernsteinsäure, Glykolsäure, Milchsäure, Äpfelsäure, Weinsäure, Citronensäure, Ascorbinsäure, α-Ketoglutarsäure, Glutaminsäure, Asparaginsäure, Maleinsäure, Hydroxymaleinsäure, Pyruvinsäure, Phenylessigsäure, Benzoesäure, p-Aminobenzoesäure, Anthranilsäure, p-Hydroxybenzoesäure, Salicylsäure, Hydroxyethansulfonsäure, Ethylensulfonsäure, Halobenzolsulfonsäure, Toluolsulfonsäure, Naphthalinsulfonsäure, Methansulfonsäure und Sulfanilsäure, umfassend
a) die Behandlung einer Verbindung der Formel: 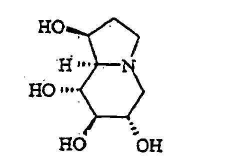 mit Bis(tributylzinn)oxid und mit einer Verbindung der Formel: 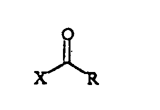 entsprechend den in Anspruch 1 angegebenen Verfahrensweisen, worin X Halogen bedeutet und R die vorstehend angegebene Bedeutung besitzt und
b) die anschließende Behandlung der Reaktionsmischung mit einem geeigneten organischen Lösungsmittel und einer Säure der vorstehend definierten Formel Y.

3. Verfahren gemäß entweder Anspruch 1 oder Anspruch 2, worin R C₁-C₄, Alkyl oder Phenyl bedeutet.

4. Verfahren gemäß entweder Anspruch 1 oder Anspruch 2, worin R für -CH₂CH₂CH₃ steht.

5. Verfahren gemäß Anspruch 4, worin das organische Lösungsmittel gemischte Xylole ist.

6. Verfahren gemäß Anspruch 5, worin Y Chlorwasserstoff bedeutet.

7. Verfahren gemäß Anspruch 6, worin X Cl bedeutet.

8. Verfahren gemäß Anspruch 7, worin die Mischung, umfassend die Verbindung der Formel, 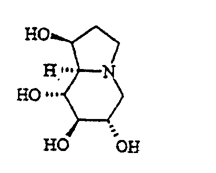 des Bis(tributylzinn)oxids und der gemischten Xylole unter Rückfluß 1 bis 6 Stunden erhitzt wird.

9. Verfahren gemäß Anspruch 8, worin die Mischung unter Rückfluß 1 bis 2 Stunden erhitzt wird.

10. Verfahren gemäß Anspruch 9, worin das geeignete organische Lösungsmittel Ethanol 3C ist.

## Revendications

1. Procédé pour la préparation d'un composé de formule : 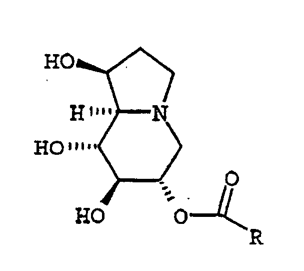 dans lequel R est un alkyle en C₁ à C₁₀, un phényle ou un phényle substitué avec un alkyle en C₁ à C₄, un nitro ou un atome d'halogène choisi dans le groupe comprenant le fluor, le chlore, le brome ou l'iode situé en position ortho, méta ou para sur le cycle,
comprenant le traitement d'un composé de formule : 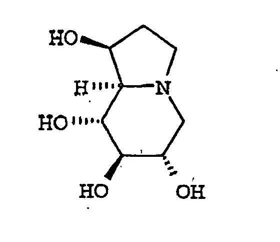 avec l'oxyde de bis (tributylétain) dans un solvant organique choisi dans le groupe comprenant le *o*-xylène, *m*-xylène, *p*-xylène et un mélange,de xylènes ;
et par la suite le traitement du mélange réactionnel avec un composé de formule : 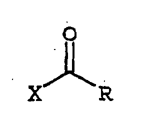 dans lequel X est un halogène et R est défini comme ci-dessus.

2. Procédé pour la préparation d'un composé de formule : 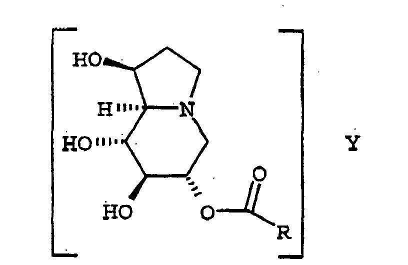 dans lequel R est un alkyle en C₁ à C₁₀, un phényle ou un phényle substitué avec un alkyle en C₁ à C₄, un nitro ou un atome d'halogène choisi dans le groupe comprenant le fluor, le chlore, le brome ou l'iode situé en position ortho, méta ou para sur le cycle ; et Y est un acide choisi dans le groupe comprenant le bromure d'hydrogène, le chlorure d'hydrogène, l'acide sulfurique, l'acide phosphorique, l'acide nitrique, l'acide formique, l'acide acétique, l'acide propionique, l'acide succinique, l'acide glycolique, l'acide lactique, l'acide malique, l'acide tartrique, l'acide citrique, l'acide ascorbique, l'acide α-cétoglutarique, l'acide glutamique, l'acide aspartique, l'acide maléique, l'acide hydroxymaléique, l'acide pyruvique, l'acide phénylacétique, l'acide benzoïque, l'acide *p*-aminobanzoïque,l'acide anthranilique, l'acide *p*-hydroxybenzoïque, l'acide salicylique, l'acide hydroxyéthanesulfonique, l'acide éthylènesulfonique, l'acide halogénobenzènesulfonique, l'acide toluènesulfonique, l'acide naphtalènesulfonique, l'acide méthanesulfonique et l'acide sulfanilique, comprenant ;
a) le traitement d'un composé de formule : 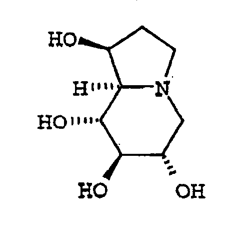 avec l'oxyde de bis (tributylétain) et avec un composé de formule : 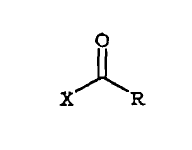 conformément aux procédures selon la revendication 1, dans lequel X est un halogène et R est défini comme ci-dessus ; et
b) par la suite le traitement du mélange réactionnel avec un solvant organique approprié et un acide de formule Y défini comme ci-dessus.

3. Procédé selon la revendication 1 ou la revendication 2 dans lequel R est un alkyle en C₁ à C₄ ou un phényle.

4. Procédé selon la revendication 1 ou la revendication 2 dans lequel R est -CH₂CH₂CH₃.

5. Procédé selon la revendication 4 dans lequel le solvant organique est un mélange de xylènes.

6. Procédé selon la revendication 5 dans lequel Y est le chlorure d'hydrogène.

7. Procédé selon la revendication 6 dans lequel X est Cl.

8. Procédé selon la revendication 7 dans lequel le mélange comprenant le composé de ladite formule, 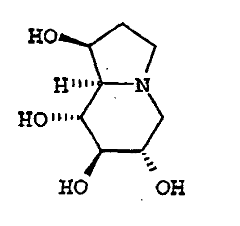 ledit oxyde de bis (tributylétain) et ledit mélange de xylènes, est chauffé à reflux pendant 1 à 6 heures.

9. Procédé selon la revendication 8 dans lequel le mélange est chauffé à reflux pendant 1 à 2 heures.

10. Procédé selon la revendication 9 dans lequel le solvant organique approprié est l'éthanol 3C.
